# EUROPEAN PATENT APPLICATION

(11) **EP 1 510 821 A1**
(43) Date of publication of application: **02.03.2005**
(21) Application number: 03077682.7
(22) Date of filing: 27.08.2003
(51) Int. Cl.: G01N 33/569, C07K 4/04, C07K 14/35

(54) **Diagnosis and treatment of mycobacterial infections**

(71) Applicant: Universiteit Utrecht Holding B.V., 3584 CK Utrecht (NL)
(72) Inventor: Koets, Adriaan Peter, 3991 GZ Houten (NL); van Eden, Willem, 3723 HD Bilthoven (NL); Rutten, Victor Pierre Marie Gerard, 3981 TS Bunnik (NL)
(74) Representative: Prins, Adrianus Willem, Mr. Ir.

(57) **Abstract**

The invention provides a method for detecting infection of an animal with a micro-organism that causes a slow progressive disease, comprising providing said animal with a protein of said micro-organism, or a functional part, derivative and/or analogue thereof, and measuring an immune response of said animal directed against said micro-organism. With a method of the invention, diagnosis during early stages of an infection has become possible. In one aspect said micro-organism comprises *Mycobacterium avium subspecies paratuberculosis*. Preferably, said protein comprises a surface-associated protein. Peptides comprising B-cell epitopes of *M.avium spp paratuberculosis* heat shock protein 70, nucleic acid molecules encoding such peptides and diagnostic kits comprising a peptide and/or nucleic acid molecule of the invention are also herewith provided.

SEQUENCE LISTING

<110>  Universiteit Utrecht Holding B.V.

<120>  Diagnosis and treatment of slow progressive infections

<130>  P62512EP00

<140>  EP 0377682.7

<141>  2003-08-27

<160>  9

<170>  PatentIn version 3.1

<210>  1

<211>  14

<212>  PRT

<213>  Mycobacterium avium paratuberculosis

<400>  1

Ile Thr Asp Ala Val Ile Thr Val Pro Ala Tyr Phe Asn Asp
1               5                   10

<210>  2

<211>  14

<212>  PRT

<213>  Mycobacterium  paratuberculosis

<400>  2

Ile Thr Asp Ala Val Ile Thr Thr Pro Ala Tyr Phe Asn Asp
1               5                   10

<210>  3

<211>  14

<212>  PRT

<213>  Escherichia coli

<400>  3

Val Thr Glu Ala Val Ile Thr Val Pro Ala Tyr Phe Asn Asp
1               5                   10

<210>  4

<211>  14

<212>  PRT

<213>  Bos taurus

<400>  4

Val Thr Asn Ala Val Val Thr Val Pro Ala Tyr Phe Asn Asp
1               5                   10

<210>  5

<211>  123

<212>  DNA

<213>  Mycobacterium avium paratuberculosis

<400>  5
cgcggccggc ggcgggtccg gatccgccga cgatgttgtg gacgcggagg tggtcgacga      60

tgaccgggag tccaagtgac acaaggaaat cagaagacgg aaggcaaccc aatccgacct     120

gca                                                                   123

<210>  6

<211>  9

<212>  PRT

<213>  Mycobacterium avium paratuberculosis

<400> 6

Pro Asp Gly Ala Ala Ala Gly Gly Gly
1               5

<210> 7

<211> 14

<212> PRT

<213> Mycobacterium avium paratuberculosis

<400> 7

Ala Gln Ala Gly Gly Pro Asp Gly Ala Ala Ala Gly Gly Gly
1               5                   10

<210> 8

<211> 14

<212> PRT

<213> Mycobacterium avium paratuberculosis

<400> 8

Pro Asp Gly Ala Ala Ala Gly Gly Gly Ser Gly Ser Ala Asp
1               5                   10

<210> 9

<211> 14

<212> PRT

<213> Mycobacterium avium paratuberculosis

<400> 9

Ala Gly Gly Gly Ser Gly Ser Ala Asp Asp Val Val Asp Ala
1               5                   10

## Description

The invention relates to the field of immunology. More specifically, the invention relates to diagnosis and treatment of slow progressive infectious diseases.

Complex cellular organisms such as mammals are often challenged by pathogenic micro-organisms. Despite many promising medical developments during the last decades infections of man, and animals such as cattle and pets, still have substantial impact on human as well as animal welfare, healthcare and food production costs. In order to provide adequate treatment, early diagnosis of infection is of utmost importance. However, a significant number of micro-organisms cause slow progressive infections that are barely, if at all, detectable in an early stage. Such latency periods often last for years. A prominent group of micro-organisms causing slow progressive infections is for instance that of the Mycobacteria e.g. *M avium, M. avium ssp. paratuberculosis, M. tuberculosis,* and *M. leprae.*

Pathogenic mycobacteria are a major cause of disease and mortality in humans as well as in many other mammalian species. In humans, tuberculosis and leprosy are major diseases worldwide, with tuberculosis alone causing over 3 million deaths each year. Similarly tuberculosis and paratuberculosis are diseases affecting, among others, economically important food producing species such as cattle. Apart from the direct economical damage at the producers level, both diseases also pose a zoonotic risk as transmission of mycobacteria via contaminated food products to humans is possible. Despite major efforts both accurate and timely diagnosis remains cumbersome and the possibilities of protecting susceptible individuals through vaccination strategies remain limited.
Most of these pathogenic mycobacteria share pathogenicity mechanisms that allow them to effectively hide from the immune system and evade a number of immune-mediated mechanisms. They share properties that enable prolonged latency periods during which they defy host-derived immune responses and consequently complicate common diagnostic approaches as well. However, to (cost)effectively eliminate these pathogens from populations at risk, accurate and timely diagnosis is essential.
As an example, paratuberculosis or Johne's disease (JD) in ruminants, caused by infection with *Mycobacterium avium subspecies paratuberculosis (M. a*. *paratuberculosis)*, leads to substantial economic losses, approx. 41 million euro annually, in the Netherlands. A serological survey on 378 dairy farms in the Netherlands revealed one or more ELISA positive cows on 55% of the farms, the true prevalence at cow level being estimated between 2 and 7% [1]. An as yet unknown number of goat and sheep flocks are infected as well, and potentially susceptible wildlife species may be involved in transmission [2]. Furthermore it has been suggested that *M. a. paratuberculosis* is involved in the aetiology of Crohn's disease in humans [3]. Thus the presence of the bacteria in the environment and food products may have human health consequences. Both animal and human health aspects justify research into the immunopathogenesis of paratuberculosis aimed at improving diagnostic tools for control of the disease and eradication strategies.
In cattle the course of the disease is as follows. Calves acquire the infection in the first months of life through oral uptake of colostrum, milk or faeces of infected cows. They either successfully clear the infection, or become subclinically infected for life. The subclinically infected animals shed the bacteria in their faeces intermittently or continuously from an age of approximately 2 years onwards. After an incubation period of 4 to 5 years, a proportion of the subclinically infected animals develops an incurable progressive form of protein losing enteropathy with chronic diarrhoea which is ultimately fatal [4].
Vaccination of calves in the first month of life prevents the development of the clinical stage of the disease, and thus reduces economical damage. However, it does not result in elimination of mycobacteria since subclinically infected animals can still be detected in approximately the same frequency in vaccinated herds as compared to non-vaccinated herds. In addition the current vaccination strategy interferes with bovine tuberculosis diagnostics. These serious drawbacks, both from an epidemiological and a human health point of view, currently limit, or even prohibit, the use of vaccination. [5]
Accurate and timely diagnosis of the infection is a major problem and the lack of it severely hampers attempts to control the disease at farm level. The gold standard of diagnosis so far is culture of bacteria from faeces of infected cattle older than 2 years. Apart from bacterial culture, specific immune responsiveness, as determined by *in vitro* IFN-γ tests [6] or the so-called absorbed JD-ELISA [7], may identify infected animals 2 to 3 years after infection, however diagnosis in animals younger than 2 years is still problematic mainly due to poor sensitivity of the current assays. None of the immunological assays are conclusive as a diagnostic tool for the whole period of the infection, as will be addressed below. [8]
Many research projects aimed at improving diagnostic procedures and relatively few efforts have been taken to unravel the immuno-pathogenesis of this disease. Several studies indicated that the major pathological effects have an immunopathological background [9]. Data, generally derived from diagnostic studies using only Purified Protein Derivative of *M*. *a*. *paratuberculosis* (PPD-P) as antigen, indicate that initial immune responses are primarily of the cell mediated type (CMI). As the disease progresses the humoral responses become increasingly apparent. The clinical stage of paratuberculosis is associated with a (complete) loss of protective, cell-mediated, responses. For these reasons none of the immunological diagnostic procedures cover the whole period of infection, moreover alternating positive and negative results for either of the assays have been obtained. [10] This change from putative protective CMI, to permissive humoral responses has recently been associated with a switch from the so called Type 1 to Type 2 T cell reactivity. [11, 12] Type 1 responses, as described for murine model systems, are thought to be effective against intracellular pathogens, such as *M*. *a*. *paratuberculosis,* by IFN-γ production that activates macrophages, cytotoxic T cells (CTL) and induces certain isotypes of antibodies. Expanding Type 2 T cells give rise to mainly humoral responses involving different isotypes of antibodies and inhibit inflammatory responses as induced by type 1 T cells. Antigen concentration, co-stimulatory molecules and micro-environmental factors, like various types of cytokines, regulate the balance between Type 1 and Type 2 responses. [13] In cattle, as compared to inbred strains of mice, the dichotomy between Type 1 and Type 2 T cells is less clear. [14]
In bovine paratuberculosis, vaccinated animals and healthy shedders have abundant (peripheral) cell mediated responses, but are not protected against infection. [15, 16] At best, these responses correlate with preventing the progression of the disease to the clinical stage, indicating a much more complex balance between host and pathogen, than the Type 1 to Type 2 shift. Alternative to a shift may be apoptotic death of T cells, rather than activation, as a consequence of interaction with infected macrophages. [17] Destruction of reactive Type 1 T cells, by apoptosis, may be a mechanism of *in vivo* switch from Type 1 to Type 2 reactivity and in this respect it is relevant to study the nature of the interactions between the *(M. a*. *paratuberculosis* infected) macrophages and responding T cells and to characterise immune responses to the different structural and secreted bacterial antigens processed and presented in different stages of the infection to elucidate the immunopathogenesis.

Despite many efforts in the field, no suitable test is available for (early) diagnosis of slow progressive infections such as mycobacterial infections. Alternating positive and negative results for several assays have been obtained in different stages of infection, and in early stages often no diagnosis is possible at all.

It is an object of the present invention to provide an improved method for detecting infection with a micro-organism that causes slow progressive disease. It is particularly an object to provide a method for diagnosis of such infections during early, latent stages. Furthermore, it is an object to at least in part counteract such infections.
Although the invention is explained in more detail for detection of mycobacterial species, it is to be understood that a method of the invention is suitable for detection of any microbial species that causes slow progressive infectious disease in cellular organisms such as mammals.

The invention provides a method for detecting infection of an animal with a micro-organism that causes a slow progressive disease, comprising providing said animal with a protein of said micro-organism, or a functional part, derivative and/or analogue thereof, and measuring an immune response of said animal directed against said micro-organism. Preferably, said micro-organism comprises a member of a mycobacterial species.
It has been found by the present inventors that diagnosis of an infection with a micro-organism that causes slow progressive disease, such as a mycobacterial species, is possible during early stages of infection if an animal is provided with a protein of said micro-organism, or with a functional part, derivative and/or analogue thereof. Said (mycobacterial) protein may be administered before, during and/or after infection. In one embodiment of the invention, a mycobacterial protein is administered before infection takes place. According to the invention, if administration of a mycobacterial protein is followed by a mycobacterial infection, such strong booster immune response is evoked in said animal that detection of said response is possible, also during early stages of said infection. Usually, a mycobacterial infection is undetectable for years, even after multiple exposures of an animal to *Mycobacteria*. Surprisingly however, after administration of a mycobacterial protein, a mycobacterial subsequent infection is capable of eliciting an immune response that is strong enough to be measured, whereas multiple exposures to mycobacteria are not capable of inducing such strong booster reactions. As shown in the examples, exposure to mycobacteria can already be demonstrated within 14 days after administration of a mycobacterial protein. While repeated exposures to mycobacteria are not capable of eliciting a booster immune response that is strong enough to be detected in early stages, a mycobacterial infection after exposure to a mycobacterial protein is capable of eliciting a measurable immune response. Therefore, administration of a mycobacterial protein is a valuable tool for diagnosis of mycobacterial infections, even during an early, latent stage.

In another embodiment, a mycobacterial protein is administered during or after a mycobacterial infection has taken place. In that case, administration of said mycobacterial protein results in a measurable immune response. A detectable immune response is obtained after administration of said protein within short.
Hence, it is possible to test animals for mycobacterial infection with a method of the invention. If an animal is already infected, a measurable immune response is obtained after administration of a mycobacterial protein. If an animal appears to be not infected (yet) with Mycobacteria, said animal can be routinely screened after certain time intervals. If said animal is infected in a later stage, a strong immune response will be elicited due to the formerly administered mycobacterial protein. Said immune response can for instance be detected during such routine screening procedure.

An animal may be directly provided with a mycobacterial protein or functional part, derivative and/or analogue thereof by administration of said mycobacterial protein or functional part, derivative and/or analogue, preferably in the presence of a suitable adjuvant such as for instance Dimethyl dioctadecyl ammonium bromide (DDA), Specol or a double oil emulsion. In another embodiment however, said animal is indirectly provided with said mycobacterial protein or functional part, derivative and/or analogue thereof, for instance by administration of a nucleic acid encoding said mycobacterial protein or functional part, derivative and/or analogue. Said nucleic acid for instance comprises DNA or RNA. Preferably said nucleic acid comprises DNA. Upon expression of said nucleic acid by the animal's machinery, mycobacterial protein or functional part, derivative and/or analogue thereof will be present and a mycobacterial infection can be detected. Said nucleic acid may be expressed in any cell type of said animal. In one embodiment, said expression only takes place in one or several specific tissue/cell types. Said nucleic acid may be artificially adapted for expression in certain kind(s) of cells only.

By a mycobacterial protein is meant a proteinaceous molecule that is involved with a *Mycobacterium.* Such protein may for instance be a part of such *Mycobacterium*. Such protein may also be a protein that is produced and/or excreted by said *Mycobacterium.* A functional part of a *Mycobacterial* protein is defined as a part which has the same kind of properties in kind, not necessarily in amount. Preferably, said functional part has the same kind of immunogenic properties, though such immunogenic properties need not be equal in amount. By immunogenic properties is meant the capability to induce an immune response in a host. A functional part of a mycobacterial protein preferably comprises an epitope of said protein. More preferably, said functional part comprises a B-cell and/or T-cell epitope of said protein.
A functional derivative of a protein is defined as a protein which has been altered such that the immunogenic properties of said derivative are essentially the same in kind, not necessarily in amount. A derivative can be provided in many ways, for instance through conservative amino acid substitution. As is known by a person skilled in the art, a substitution of one amino acid with another with generally similar properties (size, hydrophobicity, etc), does not seriously affect the overall functioning of a proteinaceous molecule.
A person skilled in the art is well able to generate analogous compounds of a protein. This can for instance be done through screening of a peptide library. Such an analogue has essentially the same immunogenic properties of said protein in kind, not necessarily in amount.
As used in this application, the term "mycobacterial protein" is meant to comprise a functional part, derivative and/or analogue of said mycobacterial protein as well.
An animal may be a non-human animal such as cattle, or a human individual. Preferably, said animal comprises a mammal. In one preferred embodiment said mammal comprises a human individual. In another preferred embodiment said mammal comprises a ruminant.

An immune response of an animal directed against a *Mycobacterium* is defined herein as a reaction of the animal's immune system against said *Mycobacterium.* Preferably, said reaction is specifically directed against said *Mycobacterium.* Said immune response preferably comprises T cell and/or B cell activation. In a preferred embodiment a method of the invention is provided wherein said immune response directed against said *Mycobacterium* comprises an immune response specifically directed against said mycobacterial protein or functional part, derivative and/or analogue thereof.

In a preferred embodiment of the invention, said immune response is measured within twelve months after said animal has been infected by a mycobacterial micro-organism. Early diagnosis facilitates the possibilities for early treatment, and for early isolation of contaminated animals and/or contaminated material from such animals in order to prevent spreading of the disease.

An animal can be provided with a mycobacterial protein in various ways. Said protein can for instance be administered with a nasal spray or orally. Alternatively, said protein can be administered via an injection, for instance subcutaneous or intramuscular. Such injection preferably comprises a suitable adjuvant such as for instance Dimethyl dioctadecyl ammonium bromide (DDA), Specol or a double oil emulsion. Said mycobacterial protein may furthermore be coupled to a suitable carrier, such as keyhole limpet hemocyanin (KLH) or an immunogenic conjugate of a protein such as ovalbumin. Other methods of providing an animal with a protein are known in the art, which may be used in a method of the invention. Preferably said protein is administered by subcutaneous injection.

In the art many protocols are known for measuring an immune response. In a preferred embodiment, (memory) B-cell activation is measured in a method of the invention. For instance, antibodies specifically directed towards mycobacteria can be detected with ELISA and/or a biosensor. In another preferred embodiment, memory T-cell activation is measured in a method of the invention. Activation of (memory) T-cells is preferably measured by using peripheral blood mononuclear cells (PBMC's) for evaluating lymphoproliferative responses with a ³H-thymidine incorporation assay. However, alternative methods are known in the art. Preferably, said antibodies and/or T-cells are specifically directed against said mycobacterial protein or functional part, derivative and/or analogue thereof.

In a preferred embodiment, a method of the invention is provided wherein said *Mycobacterium* comprises *Mycobacterium avium*. More preferably, said *Mycobacterium* comprises *Mycobacterium avium subspecies paratuberculosis.* In yet another preferred embodiment, a method of the invention is provided wherein said animal comprises a ruminant, especially cattle, sheep, goat, and/or deer. As is outlined above, mycobacterial infections in ruminants cause substantial economic losses. Early diagnosis in ruminants is therefore highly desired.

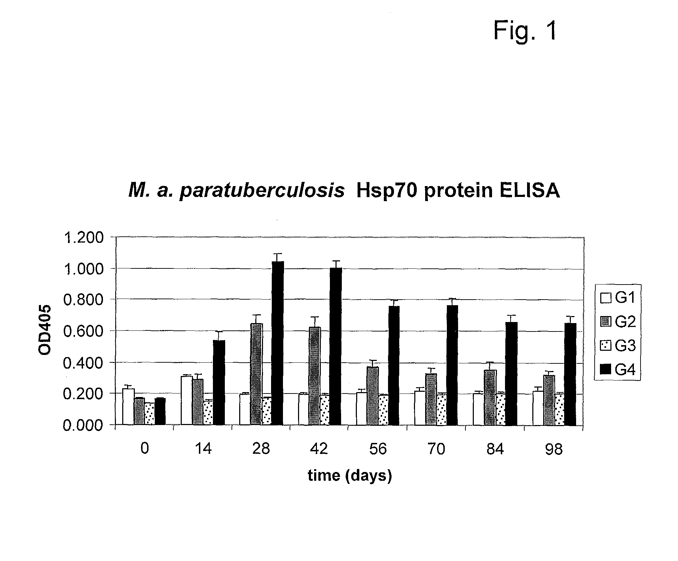

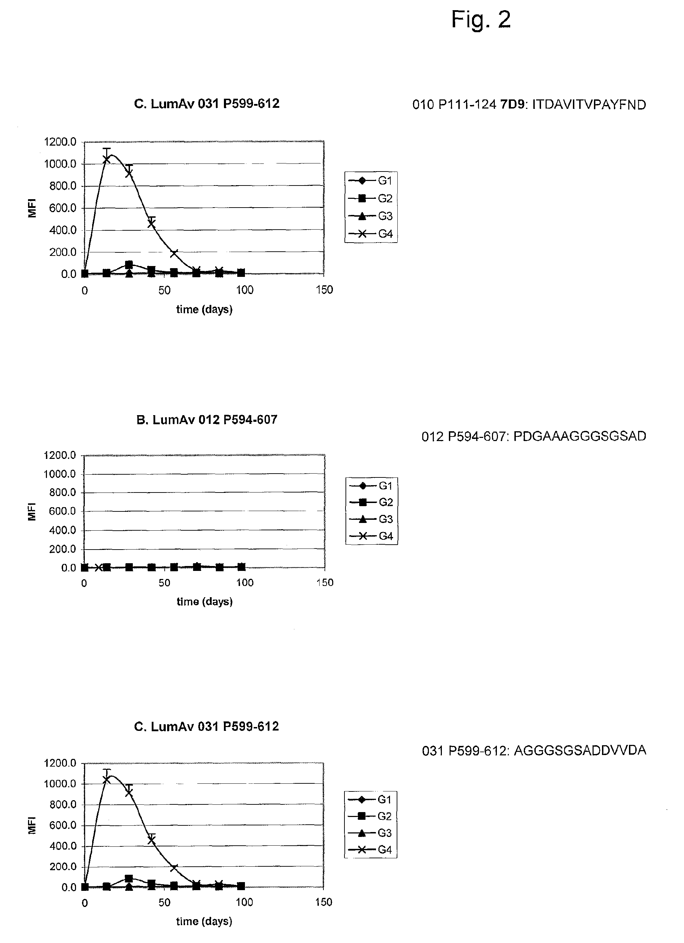

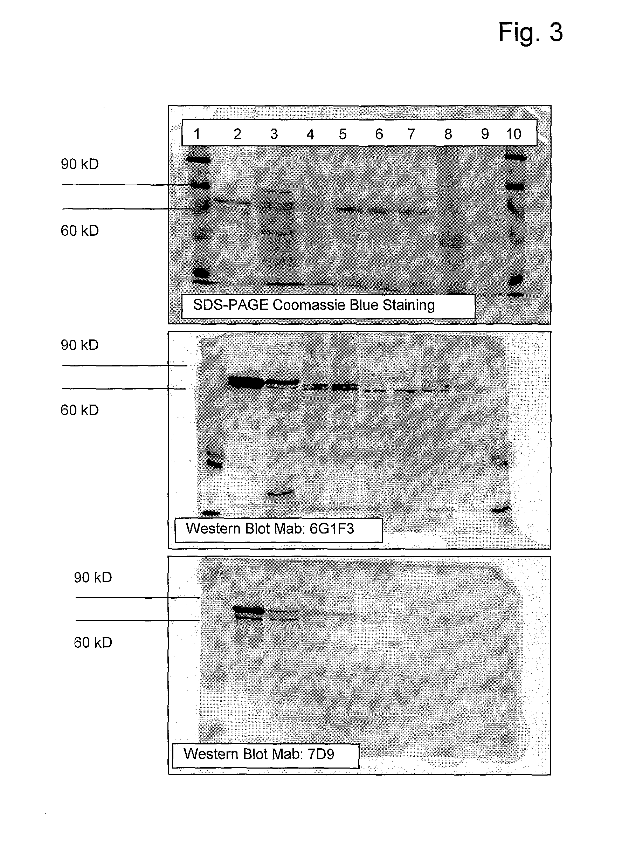

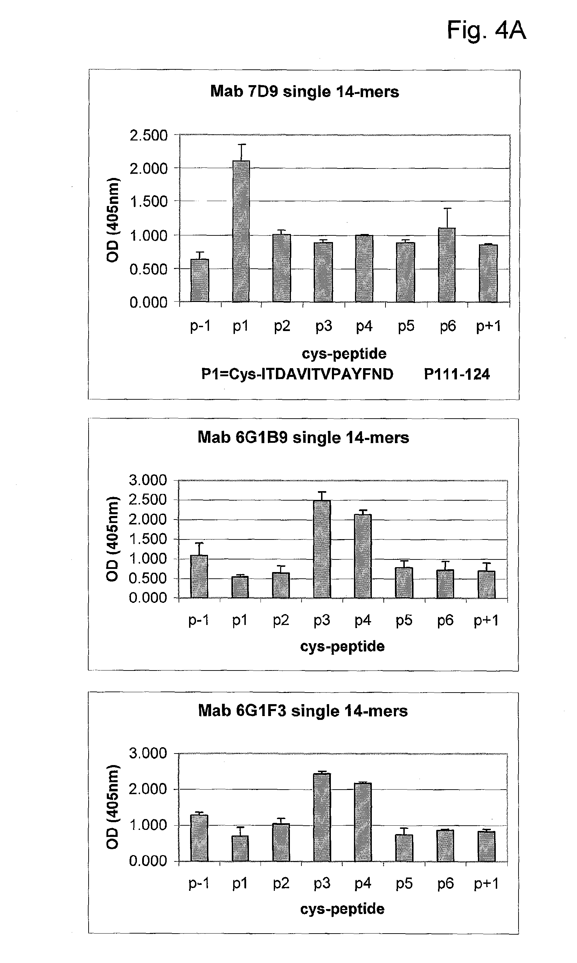

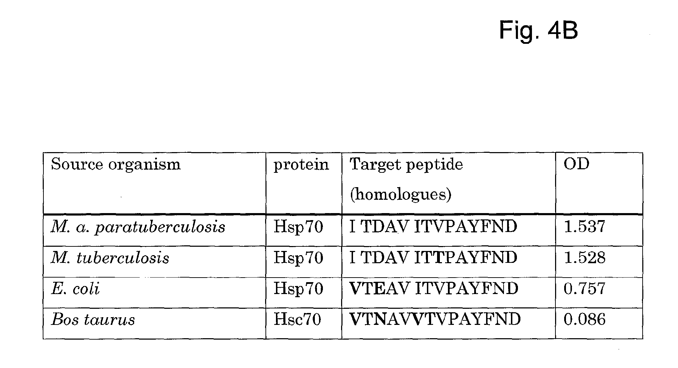

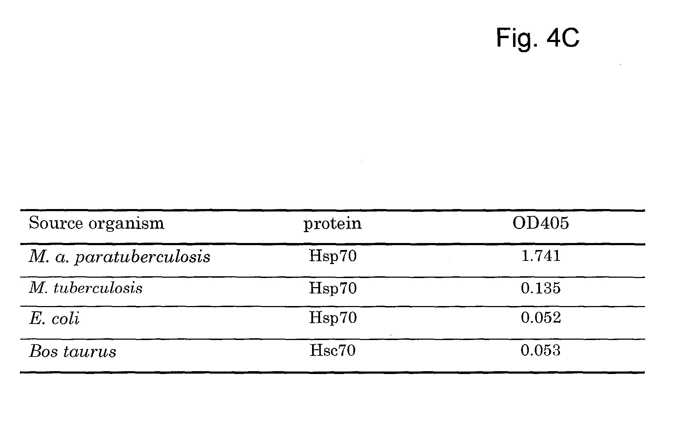

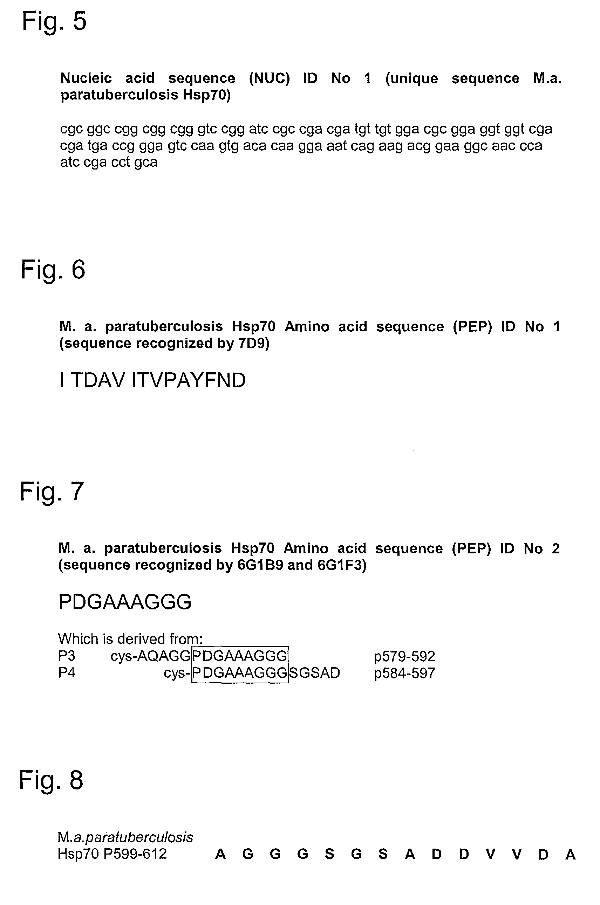

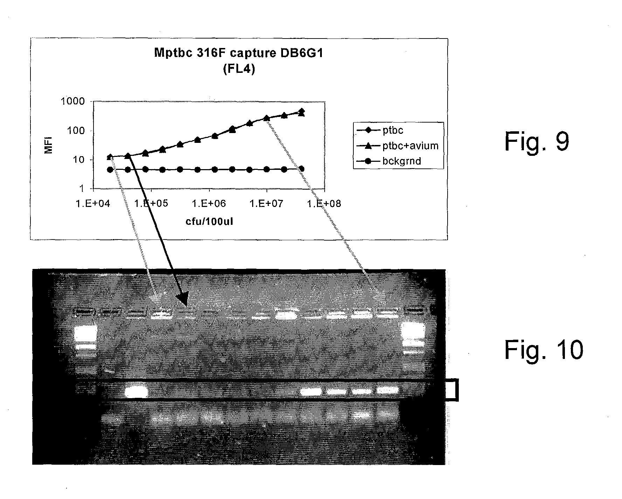

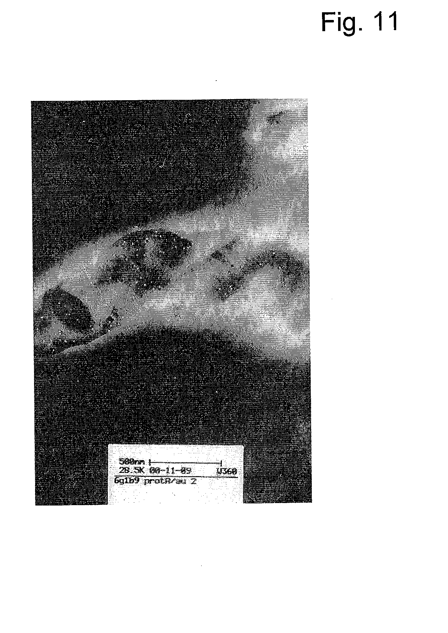

In one aspect a method of the invention is provided wherein said mycobacterial protein or functional part, derivative and/or analogue thereof comprises mycobacterial heat shock protein 70 or functional part, derivative and/or analogue thereof. Said functional part preferably comprises an epitope of said protein, such as a B-cell or T-cell epitope. More preferably, said functional part comprises a B-cell epitope. Preferred peptides comprising B-cell epitopes of the invention are depicted in figures 6, 7 and 8.
Heat shock, or stress, proteins are cytoplasmatic proteins involved in intracellular processes of protein synthesis and translocation. The expression of Hsp is upregulated during cellular stress; such as temperature, hypoxia, and tissue damage or inflammation. As such they are essential in protection of both eukaryotic and prokaryotic cellular organisms against the potentially harmful effects of stress. Hsp have been defined as immunodominant, although most of them are highly conserved and ubiquitously distributed. [19] Members of the 60, 70 and 90 kD Hsp families are involved in important aspects of bacterial infections [20], and in autoimmune diseases [21]. Hsp act as immunological target structures either by themselves because of an unusual expression pattern, or they are carrier proteins for immunogenic peptides [22]. In addition to a classical major histocompatibility complex (MHC) restricted T cell response, a major contribution in the recognition of heat shock proteins has been shown for non-MHC restricted effector cells including gamma/delta TCR positive T lymphocytes and natural killer (NK) cells. [23]
The Hsp65 and Hsp70 *of M*. *a*. *paratuberculosis* have been cloned and sequenced. [26, 27] These evolutionary conserved proteins have not been studied intensively due to doubt as to their diagnostic value in conventional serological approaches. [28] The present inventors however have studied immune responses of cattle to Hsp65 and Hsp70 extensively with regard to their role in the immunopathogenesis of bovine paratuberculosis. They have shown that both Hsp's are immunodominant antigens in bovine paratuberculosis in terms of cell mediated and antibody mediated immune responses.

According to one embodiment, early, single immunisation of young animals with recombinant 70 kD Heatshock protein (Hsp70) originating from *Mycobacterium avium ssp. paratuberculosis (M. a*. *paratuberculosis),* or with a functional part, derivative and/or analogue thereof such as for instance a peptide comprising a B-cell epitope of Hsp70, strongly primes their immune system to subsequent exposure to mycobacterial organisms. This priming, when followed by exposure to a mycobacterial pathogen induces immunological booster reactions that at least lead to activation of helper T cells and B cells and the production of antigen specific immunoglobulins. This enables rapid and sensitive diagnosis of exposure to mycobacterial organisms using available technology for the measurement of (memory) T and B cell activation.
Alternatively, immunisation with Hsp70 or a functional part, derivative and/or analogue thereof after exposure to a mycobacterial organism results in a strong immune response that can be measured. Now that a method of the invention is provided, other proteins or functional parts, derivatives and/or analogues thereof, optionally originating from different kind of micro-organisms causing a slow progressive disease, can be screened for their capability of eliciting a measurable immune response in a host after infection with said micro-organism.

In one aspect a method of the invention is provided wherein said protein or functional part, derivative and/or analogue thereof comprises a surface-associated protein or functional part, derivative and/or analogue thereof. After exposure to said micro-organism, the host's immune system is immediately exposed to such surface-associated protein. In a preferred embodiment said protein comprises a surface-associated mycobacterial protein. According to the present invention, *Mycobacterium avium subspecies paratuberculosis* heat shock protein 70 is a surface-associated mycobacterial protein and, hence, very suitable for a method of the invention. Likewise, *Mycobacterium avium* Hsp 60 is associated with the surface of *M. avium*.

In yet another preferred embodiment, said protein is recombinantly produced. This allows for controlled production of said protein. Moreover, it is possible to influence one or more properties of such protein. For instance, said protein may be rendered more immunogenic. This can for instance be done by generating a fusion protein comprising said protein and another immunogenic moiety, such as a T helper cell epitope.

In one aspect the invention provides an isolated peptide comprising a B-cell epitope of a *Mycobacterium avium subspecies paratuberculosis* heat shock protein 70 or a functional part, derivative and/or analogue thereof. A peptide comprising a B-cell epitope is particularly suitable for use in a method of the invention. It is not necessary to administer a whole mycobacterial protein to an animal because a functional part of such protein, such as a peptide comprising a B-cell epitope, is also capable of inducing a strong immune response after a mycobacterial infection. A peptide of the invention preferably varies in length from about 5 to about 500 amino acid residues. More preferably, said peptide has a length of about 5-100 amino acid residues, most preferably about 5-20 amino acid residues. Administration of a peptide of the invention instead of a whole mycobacterial protein is preferred, because undesirable side-effects of a whole mycobacterial protein can be at least partly diminished by a peptide of the invention. For instance, a part of said mycobacterial protein responsible for an adverse side-effect can be omitted in a peptide of the invention.

Moreover, a peptide of the invention is suitable for detecting *Mycobacterium-directed* antibodies. A mycobacterial infection of an animal can be detected by determining whether *Mycobacterium-directed* antibodies are present in a sample of said animal. Such antibodies can for instance be detected by common immunofluorescence, immunoblot, ELISA and/or biosensor techniques, using a peptide of the invention. As is known in the art, many alternative protocols exist for determining whether a peptide of the invention is bound to *Mycobacterium-directed* antibodies. For instance, affinity chromatography can be used.
In one aspect, a peptide of the invention is provided, wherein said epitope comprises at least a functional part of a sequence as depicted in figure 6. The sequence as depicted in figure 6 was identified by the present inventors and appears to be a linear epitope in Hsp70. This sequence is present in *M.avium* and in *M.avium spp paratuberculosis.* In another aspect a peptide of the invention is provided wherein said epitope comprises at least a functional part of a sequence as depicted in figure 7. The sequence as depicted in figure 7 is a *M.avium spp paratuberculosis* epitope. In yet another aspect a peptide of the invention is provided wherein said epitope comprises at least a functional part of a sequence as depicted in figure 8, which is a *M.avium spp paratuberculosis* epitope as well.
A peptide comprising a sequence as depicted in figure 6, 7 and/or figure 8 is especially suitable for a method of diagnosis of the invention. Mycobacterium-directed antibodies can be detected. It has therefore not only become possible to detect a mycobacterial infection in an early stage, but also to distinguish between mycobacterial species. For instance, a peptide comprising a sequence as depicted in figure 7 and/or 8 is capable of specifically demonstrating *M.avium spp paratuberculosis-directed* antibodies, whereas a peptide comprising a sequence as depicted in figure 6 is capable of demonstrating a *M.avium* infection.

A peptide comprising a sequence as depicted in figure 6, 7 and/or 8 is also suitable for eliciting antibodies against *M*.*avium*. This can be performed for vaccination purposes, for instance in human beings and in cattle. Alternatively, a non-human animal may be provided with a peptide of the invention in order to generate and collect antibodies directed against *M*.*avium*. Obtained antibodies can be used for diagnosis (demonstrating the presence of mycobacterial antigens in a sample), passive immunisation, etcetera.

Also provided is an isolated nucleic acid molecule encoding a peptide of the invention, or a functional part, derivative and/or analogue thereof. Preferably, said nucleic acid encodes a peptide varying in length from about 5 to about 500 amino acid residues. More preferably, said nucleic acid encodes a peptide varying in length from about 5 to about 100 amino acid residues, most preferably from about 5 to about 20 amino acid residues. Such isolated nucleic acid is suitable for generation of a peptide of the invention. In yet another aspect, an isolated nucleic acid molecule comprising at least a functional part of a sequence as depicted in figure 5 is provided. A nucleic acid sequence as depicted in figure 5 is particularly indicative for *M*.*avium spp paratuberculosis.* No other mycobacterial species/subtypes are known to comprise a nucleic acid sequence as depicted in figure 5. Hence, with a nucleic acid molecule comprising said sequence it has become possible to distinguish *M*.*avium spp paratuberculosis* from other mycobacterial species. It has therefore not only become possible to detect a mycobacterial infection in an early stage, but also to distinguish between mycobacterial species. For instance, a sample from a potentially infected animal can be screened for the presence of a nucleic acid molecule capable of hybridizing with a nucleic acid molecule of the invention. This can for instance be done by common Northern/Southern blot procedures, using a nucleic acid molecule of the invention as a probe. Alternatively, a PCR can be performed using (part of) a nucleic acid of the invention as a primer. Many other methods for detecting a specific nucleic acid sequence within a sample are known in the art, which need no further explication here. A nucleic acid of the invention may comprise DNA, RNA and/or an analogue thereof. In one embodiment a nucleic acid of the invention comprises peptide nucleic acid (PNA). PNA is an analogue of DNA in which the phosphodiester backbone has been replaced by a pseudo-peptide chain. PNA mimics the behaviour of DNA and binds complementary nucleic acid strands. The neutral backbone of PNA often results in stronger binding and greater specificity than normally achieved. In addition, the chemical, physical and biological properties of PNA can be exploited to produce, for instance, powerful molecular probes, biosensors and antisense agents.

A peptide of the invention can be administered to an animal in order to induce a measurable immune response after a mycobacterial infection. Peptides comprising an immunogenic part of a mycobacterial protein such as a B-cell epitope can be generated, for instance by conventional peptide synthesis techniques or with recombinant expression methods. If such peptides are administered to an animal instead of said whole mycobacterial protein, side effects can be at least partly reduced. Such peptide may for instance be less cross-reactive. Hence, in one aspect a method of the invention is provided wherein said animal is provided with a peptide or a functional part, derivative and/or analogue thereof of the invention. In one embodiment, said animal is provided with a peptide comprising at least part of a sequence as depicted in figure 7 and/or 8. Since a sequence as depicted in figure 7 and/or 8 is particularly indicative for *M.avium spp paratuberculosis,* diagnosis of this species is provided in this embodiment.
An animal can also be provided with a nucleic acid of the invention, for instance by gene therapy. A gene delivery vehicle can be used for this purpose, although many more methods are known in the art. A nucleic acid of the invention is preferably provided with a suitable promoter which enables expression of said nucleic acid sequence within said animal, more preferably in specific kind(s) of cells of said animal. After incorporation and expression of said nucleic acid, generated expression product is capable of inducing an enhanced immune response after a mycobacterial infection. Such animals can then be regularly screened. A mycobacterial infection can be detected in an early stage. Preferably said nucleic acid sequence encodes a peptide of the invention, comprising an immunogenic part of a mycobacterial protein, more preferably comprising a B-cell epitope of a mycobacterial protein. Said mycobacterial protein preferably comprises a heat shock protein. Most preferably, said nucleic acid sequence encodes a peptide of the invention comprising a B-cell epitope of *M*. *avium spp paratuberculosis* heat shock protein 70. Hsp70 epitopes are depicted in figures 6, 7 and 8. In one aspect, said nucleic acid comprises at least a functional part of a sequence as depicted in figure 5.
A gene delivery vehicle comprising a nucleic acid of the invention is also herewith provided, as well as a use of a gene delivery vehicle comprising a nucleic acid encoding a mycobacterial protein, or a functional part, derivative and/or analogue thereof, for detecting infection of a *Mycobacterium* in an animal. Preferably, said *Mycobacterium* comprises *Mycobacterium avium*, more preferably *Mycobacterium avium subspecies paratuberculosis*. A gene delivery vehicle comprising a nucleic acid of the invention can be generated using known methods in the art.

By at least a functional part of a nucleic acid of the invention is meant a part of said nucleic acid, at least 20 base pairs long, preferably at least 50 base pairs long, more preferably at least 100 base pairs long, comprising at least one characteristic (in kind not necessarily in amount) as a nucleic acid of the invention. Said characteristic preferably comprises an expression characteristic. Preferably, a functional part of a nucleic acid sequence as depicted in figure 5 is still capable of distinguishing *M*.*avium spp* *paratuberculosis* from other species. In one embodiment nucleic acid from *M.avium spp paratuberculosis* is capable of annealing to said functional part (preferably under stringent conditions) whereas nucleic acid from other *Mycobacterium* species is not. Moreover, an expression product of such functional part is preferably particularly indicative for *M.avium spp paratuberculosis*.
By at least a functional part of an epitope is meant an amino acid sequence that is capable of eliciting the same immune response in kind, not necessarily in amount, as said epitope.

In one aspect the invention provides a diagnostic kit comprising:
- a protein of a micro-organism that causes a slow progressive disease, or a functional part, derivative and/or analogue of said protein, and
- optionally, means for measuring an immune response of an animal.
The invention also provides a diagnostic kit comprising:
- a mycobacterial protein, or a functional part, derivative and/or analogue thereof, and
- optionally, means for measuring an immune response of an animal.
In another embodiment the invention provides a diagnostic kit comprising:
- a nucleic acid comprising a sequence encoding a protein of a micro-organism that causes a slow progressive disease or a functional part, derivative and/or analogue of said protein, and
- optionally, means for measuring an immune response of an animal. Preferably, said micro-organism comprises a *Mycobacterium.*
More preferably, said *Mycobacterium* comprises *Mycobacterium avium*. Most preferably said mycobacterial protein comprises a *Mycobacterium avium subspecies paratuberculosis* protein. In one embodiment a diagnostic kit of the invention is provided, wherein said mycobacterial protein comprises *Mycobacterium avium subspecies paratuberculosis* heat shock protein 70. In yet another aspect a diagnostic kit is provided comprising a peptide or a nucleic acid of the invention.
A diagnostic kit of the invention is suitable for early detection of an infection with a micro-organism that causes a slow progressive disease. In one embodiment said infection comprises a mycobacterial infection. For instance, such diagnostic kit can be used by farmers to detect *Mycobacterium avium ssp paratuberculosis* in cattle. Alternatively, a diagnostic kit of the invention can be used to detect mycobacterial infection in humans, such as infection with *M*.*tuberculosis* and *M*.*leprae*. A mycobacterial protein, a functional part, derivative and/or analogue thereof capable of inducing a measurable immune response after a mycobacterial infection, and/or a peptide of the invention can be administered to a human individual or to a non-human animal. Subsequently, an immune response can be detected if said human or animal is infected with mycobacteria. It is of course preferred to use a protein or peptide derived from the same *Mycobacterium* species that is tested for. However, since cross-reactions are possible, for instance with several *M. avium* heat shock proteins, this is not always necessary. After a mycobacterial infection is detected, it is often desirable to identify the kind of mycobacterial species. This can for instance be done with a nucleic acid molecule comprising a sequence as depicted in figure 5. As is described above, this sequence is indicative for *M*. *avium spp paratuberculosis.* Said nucleic acid molecule can therefore be used to determine whether nucleic acid from an unknown mycobacterial species is capable of annealing to said nucleic acid molecule under stringent conditions. If nucleic acid present in a sample is capable of annealing under stringent conditions, it is indicative for the presence of *M*. *avium spp paratuberculosis* nucleic acid in said sample. A peptide comprising a sequence as depicted in figure 7 and/or 8 is capable of specifically demonstrating the presence of *M*.*avium spp paratuberculosis* as well.

In order to detect mycobacterial nucleic acid in a sample, said nucleic acid often has to be amplified. Amplification can be performed with common methods in the art, such as for instance PCR, Nasba, SDA and/or TMA, which are well known in the art and need no further explanation here. An amplification reaction can be performed with non-specific primers, after which the presence of a nucleic acid of interest can be detected with a specific probe. Said amplification reaction can alternatively be performed with specific primers, or with a combination of specific and non-specific primers. In order to detect mycobacterial nucleic acid, more preferably *Mycobacterium avium spp paratuberculosis* nucleic acid, it is preferred to use at least part of a nucleic acid of the invention as a primer or probe. It is clear that, if a primer or probe is meant to anneal to a sense strand of mycobacterial nucleic acid, said primer or probe preferably comprises the anti-sense strand sequence of said mycobacterial nucleic acid sequence, and vice versa. In one aspect the invention provides a primer or probe capable of hybridizing to a nucleic acid molecule of the invention.

An isolated antibody, or a functional part, derivative and/or analogue thereof, capable of specifically binding a peptide of the invention, is also herewith provided. A functional part of an antibody is defined as a part which has essentially the same properties of said antibody in kind, not necessarily in amount. Said functional part is preferably capable of binding at least one same antigen, as compared to said antibody. However, said functional part may bind such antigen to a different extend. A functional part of an antibody for instance comprises a FAB fragment or a single chain antibody. A derivative of an antibody is defined as an antibody which has been altered such that the immunogenic properties of said antibody are essentially the same in kind, not necessarily in amount. A derivative can for instance be provided through conservative amino acid substitution. A derivative of the invention also comprises a fusion protein with essentially the same immunogenic properties of said antibody in kind, not necessarily in amount. An analogue of an antibody can for instance be found through screening of a peptide library. Such an analogue has essentially the same immunogenic properties of said antibody in kind, not necessarily in amount.
An antibody or functional part, derivative and/or analogue of the invention can be used to detect the presence of a micro-organism that causes a slow progressive disease, such as for instance a *Mycobacterium,* in a sample. Said antibody or functional part, derivative and/or analogue can for instance be coated on an ELISA microtiter plate or a biosensor, after which said plate or biosensor can be incubated with a sample from a human individual or a non-human animal such as a ruminant. If said antibody appears to have bound to some component of said sample, it is indicative for the presence of proteins, or fragments thereof, of said micro-organism in said sample and, hence, for infection with said micro-organism. Said antibody may be obtained from an infected individual. Said antibody can for instance be isolated from a sample obtained from said infected individual. Alternatively, a non-human animal can be provided with said micro-organism in order to induce an immune response. Produced antibodies can subsequently be collected from said animal. Alternatively, said antibody can be recombinantly produced, for instance by micro-organisms, cell lines or transgenic animals. Said antibody may as well be synthesized using common techniques such as solid phase synthesis. As used herein, the term "antibody" is also meant to comprise a functional part, derivative and/or analogue of said antibody.
If a Mycobacterium-directed antibody is isolated from a sample obtained from an individual, it is in itself indicative of mycobacterial infection of said individual.

A measurable immune reaction obtained with a method of the invention is not only capable of demonstrating infection. It is also capable of, at least in part, counteracting said infection. For instance, a mycobacterial infection can be, at least in part, treated by inducing an enhanced immune response in an animal with a method of the invention. In one aspect the invention therefore provides a method for at least in part treating an infection of a *Mycobacterium* in an animal, comprising providing said animal with a mycobacterial protein, or functional part, derivative and/or analogue thereof, and/or with a peptide of the invention. Preferably, said *Mycobacterium* comprises *Mycobacterium avium*, more preferably *Mycobacterium avium subspecies paratuberculosis*.
Said Mycobacterial protein preferably comprises heat shock protein 70.

A pharmaceutical composition comprising a protein or functional part, derivative and/or analogue of the invention is also herewith provided. Preferably, said protein or functional part, derivative and/or analogue comprises a mycobacterial protein or functional part, derivative and/or analogue. Said pharmaceutical composition preferably comprises a suitable adjuvant. In one embodiment said pharmaceutical composition comprises a peptide of the present invention.
In another aspect, a pharmaceutical composition comprising a nucleic acid of the invention is provided. Preferably, said nucleic acid encodes a mycobacterial protein, or functional part, derivative and/or analogue thereof. Said pharmaceutical composition can for instance be used for gene therapy, as described above.
Preferably, a pharmaceutical composition of the invention is provided, wherein said *Mycobacterium* comprises *Mycobacterium avium*. More preferably, said *Mycobacterium* comprises *Mycobacterium avium subspecies paratuberculosis.* In one aspect of the invention, said Mycobacterial protein comprises a *Mycobacterium avium subspecies paratuberculosis* heat shock protein 70 or functional part, derivative and/or analogue thereof. A method for at least in part treating an infection of an animal with a micro-organism that causes a slow progressive disease, comprising providing said animal with a pharmaceutical composition of the invention is also herewith provided. Said micro-organism preferably comprises a *Mycobacterium,* more preferably *M. avium*, most preferably *M. avium spp paratuberculosis*.

The following examples are meant to illustrate the present invention. They do not limit the scope of the invention in any way.

### Examples

### Material & Methods

### Experimental paratuberculosis infections in calves

### Animals and experimental design

A total of 40 calves (aged 29 ± 9 days at the start of the experiment) were randomly assigned to one of the following 4 experimental groups.

| Group | n | Infection | Immunisation |
|---|---|---|---|
| G1 | 10 | no | no |
| G2 | 10 | no | yes |
| G3 | 10 | yes | no |
| G4 | 10 | yes | yes |

The calves were raised using conventional procedures and feeds, and were checked daily for general health. Calves in groups 1 and 2 were physically separated from calves in groups 3 and 4, and rigorous hygienic measures were taken to prevent infection of the control groups. Body weight was recorded every 2 weeks. Blood samples were taken every 2 weeks. Fecal samples were taken 4 times during the first year of infection at t=0, t=2, t=6 and t=9 months.

### Infection

Calves assigned to groups 3 and 4 were infected orally using feces from an *M. a. paratuberculosis* infected cow which was characterised as a shedder by fecal culture of the mycobactin-J dependant and IS900 PCR positive organism. The calves received 9 dosis of 20 grams of feces during the first 21 days of the experiment at regular intervals.

### Immunisation

Calves assigned to groups 2 and 4 were immunized once at the start of the experiment (day 0). The immunisation consisted of the administration of 200 µg of recombinant *M*. *a*. *paratuberculosis* Hsp70 in dimethyl dioctadecyl ammonium bromide (DDA) (Sigma-Aldrich, D2779), subcutaneously in the dewlap. Recombinant *M*. *a*. *paratuberculosis* Hsp70 was produced as published previously. [29]

### Synthetic peptides

Based on sequence comparisons between *M. a*. *avium* Hsp70 (TIGR) and *M*. *a*. *paratuberculosis* Hsp70 (Genbank AF254578) synthetic overlapping 14-mer peptides were synthesised from the regions that are unique to *M*. *a*. *paratuberculosis* Hsp70 (see comparison in figure 12) using simultaneous multiple peptide synthesis (SMPS). One peptide from a conserved region was synthesised also. These peptides were all biotinylated using N-terminal biotinylation during SMPS.

### Serology

Serological responses to recombinant *M*. *a. paratuberculosis* Hsp70 protein were measured using an previously described ELISA technique. [29] Peptide specific serological responses were monitored using fluorescently labelled microspheres coated with avidin (LumAv beads, Luminex, USA). In total 20 uniquely labelled microspheres were incubated with one *M. a*. *paratuberculosis* Hsp70 peptide each. The beads were subsequently mixed and incubated with serum samples. Protein-A conjugated to R-PE (Prozyme, USA) was used as a reporter to detect antibodies recognizing the peptides coupled to the beads. The antibody response was analyzed in a multiplex detection system according to instructions from the manufacturer (Luminex L100, xMAP, Luminex, Tx, USA).

### Ethics

The experiments described in this study were approved by the Ethical Committee of the Utrecht University and performed according to their regulations.

### Generation of monoclonal antibodies

Monoclonal antibodies to recombinant M. paratuberculosis Hsp70 were generated as follows. Balb/c mice were immunized with 100 µg of recombinant protein in incomplete Freunds adjuvant (IFA) 3 times subcutaneously with 3 week intervals. A final booster immunization was given by intravenous administration of 100 µg Hsp70 3 days prior to sacrificing the mice. A single cell suspension was prepared from the spleen and the splenocytes were fused to the HAT sensitive SP2\0 myeloma fusion partner. Hybridoma's were generated using conventional limiting dilution techniques. Monoclonal antibodies were purified using a thiophillic agarose affinity column (AFFI-T from Kem-En-Tec) and isotyped using the Mouse Hybridoma Subtyping Kit according to the producer's manual (Roche) and screened for antigen specificity using recombinant *M. paratuberculosis* Hsp70 protein and synthetic peptides.

### Ethics

The experiments described in this study were approved by the Ethical Committee of the Utrecht University and performed according to their regulations.

### Hsp70 localisation studies

The localisation of Hsp70 in mycobacteria was studied using the following techniques.

### Mycobacteria

The mycobacteria used in these studies are life as well as dead *M*. *a*. *avium D4, M. a*. *paratuberculosis 316F*, and killed *M. bovis. M. bovis* strain 2000-683 (a generous gift from Piet Overduin, RIVM, Bilthoven, The Netherlands) were killed using heat or ethanol (96%) incubation according to established RIVM protocols, and for reasons of comparability similar methods were used for killing the other mycobacterial species.
Bacteria were harvested during the mid-logarithmic phase of growth by centrifugation and resuspended in phosphate buffered saline (PBS) at a concentration of ca 1*10¹⁰ colony forming units (cfu) per ml as determined spectrophotometrically. Before each assay, bacteria were washed three times with PBS (ELISA-assays), PBS plus 1% BSA and 0.01% sodium azide (flow cytometry)

### Flowcytometry

Suspensions of *M*. *a*. *avium* and *M*. *a*. *paratuberculosis* (both 10¹⁰ bacteria/ml in PBS) were diluted 1:100, washed three times by short centrifuging and resuspended in PBS. These suspensions were diluted 1:100 in PBS supplemented with 1% BSA and 0.01% sodium azide (both from Sigma, St. Louis, MO) and divided in volumes of 100 µl. Antibodies were added in concentrations approximately 5 µg/ml. After incubation for 25 min at room temperature (RT) and three washes with PBS and 1% BSA and 0.01% sodium azide, FITC-labelled Goat-anti-mouse was added and incubated for 25 min at RT. Following three more washes, up to 10000 bacterial cells were analysed using the FACScan (Becton-Dickinson, San Jose, CA, USA).

### Immunocapture

The Hsp70 specific monoclonal antibodies (7D9, 6G1 B9) were coupled to Dyna450 magnetic beads according to instructions of the manufacturer (Dynal), diluted in PBS in concentration of 2x10⁶ beads / ml. FITC-labelled *M*. *a*. *avium* and *M*. *a*. *paratuberculosis* were diluted 2-fold in PBS after washing and incubated with the Dyna450 beads overnights at RT. After two more immunomagnetic separations, beads with bound bacterial cells were analysed using the FACScan (Becton-Dickinson, San Jose, CA, USA). Additionally this material was used for IS900 PCR according to methods published previously.

### Electron microscopy

Bacteria (M. *a*. *paratuberculosis* and M. *a*. *avium*) are divided in volumes of 150 µl, washed three times and resuspended in 100 µl block buffer (Roche). One µg of a 100 µg/ml antibody solution was added and all were incubated over-the-top for half an hour. Bacteria are washed 3 times with phosphate buffered saline comtaining 0,1% Tween 20 and resuspended in block buffer. Then protein A - gold (10nm) was added (1:400) and again incubated 30 min over-the-top at room temperature. Bacteria were washed as previously, and resuspended in 100 µl block buffer.
A 5 µl droplet of bacterial suspension was overlaid with a negative-stain immuno electron microscopy Ni- or Cu-grid for 15 min. The grid was rinsed three times 5 min on phosphate buffered saline containing 50 mM glycine. Next, the grid was placed on incubation buffer (0.1% acetylated bovine serum albumin in PBS) for 3 times 10 min and then washed 4 times 5 min using redistilled water. Grids were blotted dry briefly on filter paper, tipped on 5 µl potassium phosphotungstate solution (2%) and blotted completely dry on filter paper. Finally, preparates were viewed using a transmission electron microscope (Philips, Eindhoven, The Netherlands).

### ELISA using protein or bacteria

Protein antigens used were *M*. *a. paratuberculosis* Hsp70 (produced as described above), and *M. tuberculosis* Hsp70, *E. coli* Hsp70 and *Bos Taurus* Hsc70 (all from Stressgen, Vancouver, Canada) Next, 96-well plates (Corning Costar) were coated with 1 µg of antigen or 100 µl of washed bacteria, diluted in sodium bicarbonate buffer for 30 (antigens) or 60 (bacteria) min at room temperature, while shaking at 300 rpm on a electronic IKA MTS shaker (IKA). All subsequent incubations were performed for 30 min shaking at room temperature. After each incubation step plates were washed three times with phosphate buffered saline containing 0,01% Tween 20. Wells were blocked with 200 µl of Post Coating Buffer (PCB) (Roche Diagnostics GmbH, Mannheim, Germany), containing 50 mM Tris-HCl, 150 mM sodium chloride and a 1% (w/v) protein mixture that was obtained by proteolytic degradation of purified gelatin, at pH 7.4. Sera or pure antibody-solutions were (serially) diluted (see results) in PCB; this was followed by incubation Goat anti Mouse (GAM)-PO (ELISA subtyping kit, Roche) 1:2000 diluted in PCB. Finally 100 µl ABTS substrate buffer (Boehringer) was added to each well. The OD 405 nm was measured after 10 minutes using an automated ELISA reader (Biorad).

### Synthetic peptide ELISA

A 96-well CovaLink NH F8 plate (Nunc) was coated with 100 µl of 0,5 mM SPDP in 2-propanol, diluted in phosphate buffered saline (PBS) for 30 min at 37 °C. After incubation, the plate was washed two times with redistilled water (MilliQ). All reagents were transferred at 100 µl/well and all subsequent incubations were performed at 37 °C. The different cystein-linked peptides were diluted in 0.1 M Tris-HCl, pH 8.0 at a concentration of 15 µg/ml, directly before transfer to wells. Peptides were incubated during 60 min. The plate was washed as described previously. After all subsequent incubations, the plate was washed three times with tap water. Wells were blocked with 200 µl of PCB (Roche) for 15 min. Antibody-solutions were diluted to a concentration of 1 µg/ml µg/ml in PCB; this was followed by incubation with GAM-PO (ELISA subtyping kit, Roche) 1:2000 diluted in PCB. Finally 100 µl ABTS substrate buffer (Boehringer) was added to each well. The OD 405 nm was measured after 10 minutes using an automated ELISA reader (Biorad).

### Brief description of the drawings

### Figure 1

### Hsp70 specific antibody responses of calves experimentally infected with M. a. paratuberculosis

The whole protein Hsp70 ELISA demonstrates that a difference exists between the serum antibody responses of the calves in group 2 (immunisation, no infection), and group 4 (immunisation and infection). Non of the (un-)infected controls (group 1 and 3) show a response to Hsp70 in this stage of the infection.

### Figure 2

### Hsp70 peptide specific antibody responses of calves experimentally infected with M. a. paratuberculosis

Antibody responses of calves, as measured by Luminex technology (response indicated as mean fluorescence index (MFI) on the Y axis), to selected synthetic peptides from *M*. *a. paratuberculosis* Hsp70. Panel A shows the response to a conserved part of the protein, which is present in many (myco)bacteria, demonstrating booster-effects by environmental bacterial species. Panels B and C show responses to peptides which are unique to *M. a*. *paratuberculosis* Hsp70. No response was observed to the peptide shown in B, in contrast a clear response is shown in panel C, however only by calves (G4) primed with *M*. *a*. *paratuberculosis* Hsp70 and subsequently exposed to the bacteria, thus demonstrating the diagnostic capabilities of the system.

### Figure 3

### Recombinant protein production and recognition by monoclonal antibodies 7D9, 6G1B9, and 6G1F3 in western blot.

Lane 1 protein size Marker
Lane 2 purified recombinant Hsp70 protein
Lane 3 soluble protein fraction E. Coli Top 10 production strain
Lane 4 through 9 intermediate purification samples
Lane 10 protein size marker

### Figure 4

### Protein and Peptide ELISA of the M. a. paratuberculosis Hsp70 recognizing monoclonal antibodies: epitope identification.

### Figure 4A:

Peptide ELISA using, 5 amino acids overlapping, peptides of the *M. a. paratuberculosis* Hsp70 protein covering less-conserved parts of the protein. These peptides were N-terminally linked to a cysteine to enable coupling to covalink ELISA plates. Peptides were first tested in pools, subsequently positive pools were retested using the individual peptides to identify linear epitopes.

### Figure 4B:

### Protein ELISA using 7D9

Testing homologues of *M*. *a*. *paratuberculosis* Hsp70 in whole protein ELISA using 7D9 results in the following cross reactive responses.

### Figure 4C:

### Protein ELISA using 6G1B9

(3 homologues of *M*. *a*. *paratuberculosis* Hsp70, no known equivalent of the 6G1B9 target peptide in the sequence, BLAST of peptide sequence returns only M. a. paratuberculosis sequence)

### Figure 5

NUC ID No 1 (unique C-terminal sequence *M*. *a*. *paratuberculosis* Hsp70)

### Figure 6

PEP ID No 1 (sequence recognized by monoclonal 7D9)

### Figure 7

PEP ID No 2 (sequence recognised by monoclonals 6G1B9 and 6G1F3)

### Figure 8

PEP ID No 3 (sequence recognised by bovine B cells from *M. a. paratuberculosis* infected calves treated with a single Hsp70 immunisation)

### Figure 9

Flowcytometric detection of intact *M. a. paratuberculosis* using the monoclonal antibodies 7D9, 6G1B9, and 6G1F3

### Figure 10

Isolation of *M. a. paratuberculosis* using magnetic beads coated with 6G1B9 antibodies, confirmed using genomic insertion sequence IS900 specific PCR.

### Figure 11

Electronmicrographs showing surface binding of 6G1B9 using immunogold labeling

### References

1. Muskens, J., et al., *Prevalence and regional distribution of paratuberculosis in dairy herds in The Netherlands.* Vet Microbiol, 2000. **77**(3-4): p. 253-61.
2. Beard, P.M., et al., *Paratuberculosis infection of nonruminant wildlife in Scotland.* J Clin Microbiol, 2001. **39**(4): p. 1517-21.
3. Badiola, J.J., et al., *Possible links between Crohn's Disease and Paratuberculosis.* 2000, European Union: Brussel. p. 1-76.
4. Clarke, C.J., *The pathology and pathogenesis of paratuberculosis in ruminants and other species.* J Comp Pathol, 1997. **116**(3): p. 217-61.
5. Kalis, C.H., et al., *Use of long-term vaccination with a killed vaccine to prevent fecal shedding of Mycobacterium avium subsp paratuberculosis in dairy herds.* Am J Vet Res, 2001. **62**(2): p. 270-4.
6. Rothel, J., et al., *A sandwich enzyme immunoassay for bovine interferon-g and its use for the detection of tuberculosis in cattle.* Australian Veterinary Journal, 1990. **67**(4): p. 134-137.
7. Milner, A.R., et al., *The sensitivity and specificity of a modified ELISA for the diagnosis of Johne's disease from a field trial in cattle.* Vet Microbiol, 1990. **25**(2-3): p. 193-8.
8. McDonald, W.L., et al., *Evaluation of diagnostic tests for Johne's disease in young cattle.* Aust Vet J, 1999. **77**(2): p. 113-9.
9. Merkal, R.S., K.E. Kopecky, and A.B. Larsen, *Immunologic mechanisms in bovine paratuberculosis.* Am J Vet Res, 1970. **31**(3): p. 475-85.
10. Collins, M.T., *Diagnosis of paratuberculosis.* Vet Clin North Am Food Anim Pract, 1996. **12**(2): p. 357-71.
11. Chiodini, R.J., *Immunology: resistance to paratuberculosis.* Vet Clin North Am Food Anim Pract, 1996. **12**(2): p. 313-43.
12. Sweeney, R.W., et al., *Interferon-gamma and interleukin 4 gene expression in cows infected with Mycobacterium paratuberculosis.* Am J Vet Res, 1998. **59**(7): p. 842-7.
13. Abbas, A., K. Murphy, and A. Sher, *Functional diversity of helper T lymphocytes.* Nature, 1996. **383:** p. 787-93.
14. Estes, D.M. and W.C. Brown, *Type 1 and type 2 responses in regulation of Ig isotype expression in cattle.* Veterinary Immunology and Immunopathology, 2002. **90**(1-2): p. 1-10.
15. Koets, A.P., et al., *Heat-shock protein-specific T-cell responses in various stages of bovine paratuberculosis.* Vet Immunol Immunopathol, 1999. **70**(1-2): p. 105-15.
16. Wentink, G.H., et al., *Incidence of paratuberculosis after vaccination against M. paratuberculosis in two infected dairy herds.* Zentralbl Veterinarmed [B], 1994. **41**(7-8): p. 517-22.
17. Koets, A., et al., *Progressive bovine paratuberculosis is associated with local loss of CD4(+) T cells, increased frequency of gamma delta T cells, and related changes in T-cell function.* Infect Immun, 2002. **70**(7): p. 3856-64.
18. Koets, A.P., et al., *Lewis rats are not susceptible to oral infection with Mycobacterium avium subsp. paratuberculosis.* Vet Microbiol, 2000. **77**(3-4): p. 487-495.
19. Young, R., *Stress proteins and immunology.* Annu. Rev. Immunol., 1990. **8:** p. 401-20.
20. Zugel, U. and S.H. Kaufmann, *Role of heat shock proteins in protection from and pathogenesis of infectious diseases.* Clin Microbiol Rev, 1999. **12**(1): p. 19-39.
21. van Eden, W., et al., *Cloning of the mycobacterial epitope recognized by T lymphocytes in adjuvant arthritis*. Nature, 1988. **331**(6152): p. 171-173.
22. Suzue, K. and R.A. Young, *Heat shock proteins as immunological careers and vaccines.* Exs, 1996. **77:** p. 451-65.
23. Munk, M.E. and M. Emoto, *Functions of T-cell subsets and cytokines in mycobacterial infections.* Eur Respir J Suppl, 1995. **20**: p. 668s-675s.
24. Bonato, V.L., et al., *Identification and characterization of protective T cells in hsp65 DNA- vaccinated and Mycobacterium tuberculosis-infected mice.* Infect Immun, 1998. **66**(1): p. 169-75.
25. Lowrie, D., et al., *Protection against tuberculosis by a plasmid DNA vaccin*. Vaccine, 1997. **15**(8): p. 834-838.
26. Colston, A., I. McConnell, and R. Bujdoso, *Cloning and expression in Escherichia coli of DNA encoding a 60 kDa stress protein of Mycobacterium paratuberculosis, the causative agent of Johne's disease.* Microbiology, 1994. **140**: p. 3329-3336.
27. Stevenson, K., et al., *Complete nucleotide sequence of a gene encoding the 70 kd heat shock protein of Mycobacterium paratuberculosis.* Nucleic Acids Res, 1991. **19**(16): p. 4552.
28. el-Zaatari, F.A., et al., *Nucleotide sequence analysis and seroreactivities of the 65K heat shock protein from Mycobacterium paratuberculosis.* Clin Diagn Lab Immunol, 1995. **2**(6): p. 657-64.
29. Koets, A.P., et al., *Differential changes in heat shock protein-, lipoarabinomannan-, and purified protein derivative-specific immunoglobulin G1 and G2 isotype responses during bovine Mycobacterium avium subsp. paratuberculosis infection.* Infect Immun, 2001. **69**(3): p. 1492-8.
30. Vary, P.H., et al., *Use of highly specific DNA probes and the polymerase chain reaction to detect Mycobacterium paratuberculosis in Johne's disease.* J Clin Microbiol, 1990. **28**(5): p. 933-7.

## Claims

1. A method for detecting infection of an animal with a *Mycobacterium*, comprising providing said animal with a *Mycobacterial* protein, or functional part, derivative and/or analogue thereof, and measuring an immune response of said animal directed against said *Mycobacterium*.

2. A method according to claim 1, wherein said *Mycobacterial* protein, or functional part, derivative and/or analogue thereof, is provided before said animal has been infected by said *Mycobacterium*.

3. A method according to claim 1 or 2, wherein said immune response is measured within twelve months after said animal has been infected by said *Mycobacterium*.

4. A method according to any one of claims 1-3, wherein said immune response is measured within 14 days after said animal has been provided with said *Mycobacterial* protein or functional part, derivative and/or analogue thereof.

5. A method according to any one of claims 1-4, wherein said measurement of an immune response of said animal comprises a measurement of memory T-cell activation.

6. A method according to any one of claims 1-5, wherein said measurement of an immune response of said animal comprises a measurement of memory B-cell activation.

7. A method according to any one of claims 1-6, wherein said *Mycobacterium* comprises *Mycobacterium avium*.

8. A method according to any one of claims 1-7, wherein said *Mycobacterium* comprises *Mycobacterium avium subspecies paratuberculosis.*

9. A method according to any one of claims 1-8, wherein said *Mycobacterial* protein or functional part, derivative and/or analogue thereof, comprises a surface-associated *Mycobacterial* protein or functional part, derivative and/or analogue thereof.

10. A method according to any one of claims 1-9, wherein said *Mycobacterial* protein or functional part, derivative and/or analogue thereof comprises *Mycobacterial* heat shock protein 70 or functional part, derivative and/or analogue thereof.

11. A method according to any one of claims 1-10, wherein said *Mycobacterial* protein or functional part, derivative and/or analogue thereof comprises a recombinantly produced *Mycobacterial* protein or functional part, derivative and/or analogue thereof.

12. A method according to any one of claims 1-11, wherein said animal comprises a ruminant.

13. An isolated peptide comprising a B-cell epitope of a *Mycobacterium avium subspecies paratuberculosis* heat shock protein 70 or a functional part, derivative and/or analogue thereof.

14. A peptide according to claim 13, wherein said epitope comprises at least a functional part of a sequence as depicted in figure 6.

15. A peptide according to claim 13, wherein said epitope comprises at least a functional part of a sequence as depicted in figure 7.

16. A peptide according to claim 13, wherein said epitope comprises at least a functional part of a sequence as depicted in figure 8.

17. An isolated nucleic acid molecule encoding a peptide according to any one of claims 13-16, or a functional part, derivative and/or analogue thereof.

18. An isolated nucleic acid molecule comprising at least a functional part of a sequence as depicted in figure 5.

19. A method according to any one of claims 1-12, wherein said animal is provided with a peptide or a functional part, derivative and/or analogue thereof according to anyone of claims 13-16.

20. A method according to any one of claims 1-12 or 19, wherein said animal is provided with a nucleic acid according to claim 17 or 18.

21. A diagnostic kit comprising:
- *a Mycobacterial* protein, or a functional part, derivative and/or analogue thereof, and
- optionally, means for measuring an immune response of an animal.

22. A diagnostic kit comprising:
- a nucleic acid comprising a sequence encoding a *Mycobacterial* protein, or a functional part, derivative and/or analogue thereof, and
- optionally, means for measuring an immune response of an animal.

23. A diagnostic kit according to claim 21 or 22, wherein said *Mycobacterium* comprises *Mycobacterium avium*.

24. A diagnostic kit according to any one of claims 21-23, wherein said *Mycobacterial* protein comprises a *Mycobacterium avium subspecies paratuberculosis* protein.

25. A diagnostic kit according to any one of claims 21-24, wherein said *Mycobacterial* protein comprises a *Mycobacterium avium subspecies paratuberculosis* heat shock protein 70.

26. A diagnostic kit comprising a peptide according to any one of claims 13-16 or a nucleic acid according to claim 17 or 18.

27. A primer or probe capable of hybridizing to a nucleic acid according to claim 17 or 18.

28. Use of a gene delivery vehicle comprising a nucleic acid encoding a *Mycobacterial* protein, or a functional part, derivative and/or analogue thereof, for detecting infection of a *Mycobacterium* in an animal.

29. Use according to claim 28, wherein said *Mycobacterium* comprises *Mycobacterium avium*.

30. Use according to claim 28 or 29, wherein said *Mycobacterium* comprises *Mycobacterium avium subspecies paratuberculosis.*

31. A gene delivery vehicle comprising a nucleic acid according to claim 17 or 18.

32. An isolated antibody, or a functional part, derivative and/or analogue thereof, capable of specifically binding a peptide according to any one of claims 13-16.

33. A method for at least in part treating an infection of a *Mycobacterium* in an animal, comprising providing said animal with a *Mycobacterial* protein, or functional part, derivative and/or analogue thereof.

34. A method according to claim 33, wherein said *Mycobacterium* comprises *Mycobacterium avium*.

35. A method according to claim 33 or 34, wherein said *Mycobacterium* comprises *Mycobacterium avium subspecies paratuberculosis.*

36. A method according to any one of claims 33-35, wherein said *Mycobacterial* protein comprises heat shock protein 70.

37. A pharmaceutical composition comprising a *Mycobacterial* protein, or functional part, derivative and/or analogue thereof.

38. A pharmaceutical composition comprising a nucleic acid encoding a *Mycobacterial* protein, or functional part, derivative and/or analogue thereof.

39. A pharmaceutical composition according to claim 37 or 38, wherein said *Mycobacterium* comprises *Mycobacterium avium*.

40. A pharmaceutical composition according to any one of claims 37-39, wherein said *Mycobacterium* comprises *Mycobacterium avium subspecies paratuberculosis*.

41. A pharmaceutical composition according to any one of claims 37-40, wherein said protein comprises a *Mycobacterium avium subspecies paratuberculosis* heat shock protein 70 or functional part, derivative and/or analogue thereof.

42. A method for at least in part treating a mycobacterial infection of an animal comprising providing said animal with a pharmaceutical composition according to any one of claims 37-41.
